# EUROPÄISCHE PATENTSCHRIFT

(11) **EP 0 570 604 B1**
(45) Veröffentlichungstag und Bekanntmachung des Hinweises auf die Patenterteilung: **20.09.2000**
(21) Anmeldenummer: 92107995.0
(22) Anmeldetag: 12.05.1992
(51) Int. Cl.: A61M 25/01

(54) **Elektrodenkatheter mit einer Anordung zum intravaskulären Implantieren oder Explantieren des Elektrodenkatheters**
Electrode catheter with device to intravascular placing or removing the electrode catheter
Cathéter à électrode avec dispositif pour implantation ou extraction intravasculaire du cathéter

(43) Veröffentlichungstag der Anmeldung: 24.11.1993
(73) Patentinhaber: Pacesetter AB, 171 95 Solna (SE)
(72) Erfinder: Nyman, Per, S-182 64 DJURSHOLM (SE)
(74) Vertreter: Lettström, Richard Wilhelm

(56) Entgegenhaltungen:
- EP-A- 0 267 539
- EP-A- 0 310 295
- US-A- 4 907 572
- US-A- 5 041 124

## Beschreibung

Die Erfindung betrifft einen Elektrodenkatheter mit einer Anordnung zum intravaskulären Implantieren oder Explantieren des Elektrodenkatheters, an dem eine Antriebsvorrichtung angeordnet ist, die den Katheter im Bereich seines distalen Endes in eine oszillierende Bewegung versetzt.

Das Einführen eines intravaskulären Elektrodenkatheters, beispielsweise einer Herzschrittmacherelektrode in den Körper eines Patienten wird durch Reibung zwischen Katheter und der Wandung der den Katheter aufnehmenden Blutgefäße behindert. Um die Reibung zu verringern, ist es bekannt, den Elektrodenkatheter mit einem Gleitmittel zu beschichten.

Innerhalb von einigen Monaten nach der Implantation wird der Elektrodenkatheter von einer Bindegewebsschicht eingeschlossen, die den Elektrodenkatheter derart verankert, daß es schwierig, gefährlich oder sogar unmöglich ist, den Katheter aus den ihn aufnehmenden Blutgefäßen herauszuziehen. Wenn daher eine Explantation des Katheters nicht unbedingt notwendig ist, ist es erforderlich, die mit der Explantation des Katheters verbundenen Risiken gegenüber dem Risiko für den Patienten bei einem Belassen des Katheters in seinem Körper abzuwägen. Ist dagegen wie im Falle einer Infektion eine Explantation des Katheters unbedingt erforderlich und das mit einem Herausziehen des Katheters verbundene Risiko zu groß, so müssen zum Entfernen des Katheters die ihn aufnehmenden Blutgefäße aufgeschnitten werden.

Aus der US-A-4 907 572 ist ein Ureterkatheter bereits bekannt, der die Passage eines Steins von dem Harnleiter erleichtern soll. Der Katheter wird in den Harnleiter eingeführt und durch einen vibrierenden Kopf dazu gebracht abwechselnd radial und axial zu expandieren und dadurch die Wand des Harnleiters bis zu 3 cm vom Kopf zum Oszillieren zu bringen. Der Katheter kann auf diese Weise den Stein vor sich und zurück in die Harnblase schieben.

Aus der US-A-4 749 376 ist ein intravaskulärer Katheter bekannt, der an seinem distalen Ende ein Werkzeug zur Beseitigung von Ablagerungen in Blutgefäßen oder zum Aufweiten von Gefäßverengungen aufweist. Am proximalen Ende des bekannten Katheters ist ein Motor angeordnet, dessen Rotationsbewegung durch einen Steuerdraht im Inneren des Katheters zu dessen distalen Ende übertragen wird und dort in eine oszillierende Hubbewegung des Werkzeuges in Richtung der Längsausdehnung des Katheters umgesetzt wird.

Bei einem anderen, aus der US-A-3 352 303 bekannten intravaskulären Katheter ist an dessen proximalen Ende ein elektromechanischer Wandler angeordnet, dessen Vibrationsenergie beispielsweise über einen Draht oder eine Flüssigkeit an das distale Ende des Katheters zur Auflösung von intravaskulären Blutgerinnungen übertragen wird.

Aus der US-A-4 854 325 ist ein ähnlicher Katheter zur Beseitigung von Gefäßverstopfungen bekannt, in dem ein am distalen Ende des Katheters aus diesem herausragender Steuerdraht verläuft, der durch einen Motor am proximalen Ende des Katheters in eine oszillierende Hubbewegung versetzt wird.

Den obengenannten bekannten Kathetern ist gemeinsam, daß jeweils an ihrem distalen Ende eine Hubbewegung in Richtung der Längsausdehnung des Katheters erzeugt wird, so daß Hindernisse im Einführungsweg des Katheters beseitigt werden können. Dagegen wird bei den bekannten Kathetern die Reibung zwischen den Katheter und der Wandung der den Katheter aufnehmenden Blutgefäße durch die Hubbewegungen nicht verringert. Außerdem besteht bei den bekannten Kathetern die Gefahr, daß die Wand des den jeweiligen Katheter aufnehmenden Blutgefäßes von der distalen Katheterspitze durchstoßen wird. Schließlich sind die obengenannten Katheter nicht für eine langerfristige Implantation im menschlichen Körper vorgesehen, so daß die oben angegebenen Probleme im Zusammenhang mit der Explantation des Katheters nicht auftreten.

Der Erfindung liegt die Aufgabe zugrunde, die Implantation und Explantation eines intravaskulären Elektrodenkatheters zu erleichtern, wobei gleichzeitig die Gefahr einer Schädigung der den Katheter aufnehmenden Blutgefäße verringert wird.

Gemäß der Erfindung wird die Aufgabe durch einen Elektrodenkatheter mit einer Anordnung zum intravaskulären Implantieren oder Explantieren des Elektrodenkatheters, an dem eine Antriebsvorrichtung angeordnet ist, die den Elektrodenkatheter in eine oszillierende Bewegung versetzt und der durch Mittel zur Erzeugung der oszillierende Bewegung nicht in Richtung der Längsausdehnung des Elektrodenkatheters sondern quer dazu gekennzeichnet ist, gelöst.

Dadurch, daß der Katheter im Bereich seines distalen Endes in eine oszillierende Bewegung quer zu seiner Längsausdehnung versetzt wird, wird der Katheter ständig von der ihn umgebenden Gefäßwand abgestoßen, so daß er bei einer ausreichend hohen Frequenz der oszillierenden Bewegung quasi auf der Gefäßwand schwimmt. Dadurch wird die Reibung zwischen dem Katheter und der ihn umgebenden Gefäßwand erheblich herabgesetzt, so daß sich der Katheter beim Implantieren leicht durch die in aufnehmenden Blutgefäße hindurchschieben läßt. In entsprechender Weise wird auch die Explantation des Katheters erleichtert, weil durch die oszillierende Bewegung die Verankerung des Katheters in dem ihn umgebenden Gewebe gelockert und beim Herausziehen des Katheters die Reibung verringert wird. Sowohl bei Implantieren als auch beim Explantieren des erfindungsgemäßen Katheters ist die Gefahr einer Beschädigung oder gar eines Durchstoßens der Blutgefäße minimiert, da die oszillierende Bewegung nicht in Richtung der Längsausdehnung des Katheters sondern quer dazu erfolgt.

Entsprechend einer vorteilhaften Weiterbildung des Erfindungsgemäßen Elektrodenkatheters ist vorgesehen, daß die Anordnung Mittel zur Beaufschlagung weitere Bereiche des Katheters zwischen seinem distalen Ende und seinem proximalen Ende mit der oszillierenden Bewegung umfasst. Dadurch wird nicht nur im Bereich des distalen Endes des Katheters, sondern auch entlang des Katheters die Reibung zwischen diesem und den ihn aufnehmenden Blutgefäßen verringert.

Als besonders vorteilhaft erweist sich der erfindungsgemäßen Elektrodenkatheter, weil derartige intravaskuläre Elektrodenkatheter beispielsweise zur Herzschritteraphie oder zur Defibrillation des Herzens als Dauerimplantate in den menschlichen Körper eingeführt werden und sich deswegen die eingangs beschriebenen Probleme im Falle einer Explantation des Katheters ergeben, die auf so einfache und effektive Weise mit dem erfindungsgemäßen Katheter gelöst werden.

Bei der erfindungsgemäßen Elektrodenkatheter mit einer Anordnung zum Implantieren oder Explantieren des Katheters ist entsprechend einer vorteilhaften Ausbildung vorgesehen, daß die Antriebsvorrichtung aus einem im Inneren des Katheters angeordneten elektromechanischen Wandler besteht. Dabei wird der elektromechanische Wandler durch elektrische Ansteuerung in eine oszillierende Bewegung versetzt, die an der Stelle bzw. an den Stellen, an denen der elektromechanische Wandler im Inneren des Katheters angeordnet ist, auf diesen übertragen wird.

Der elektromechanische Wandler kann integraler Bestandteil des Katheters sein oder, ohne daß dazu ein besonderer Aufbau des Katheters erforderlich ist, in vorteilhafter Weise an einem Steuerdraht angeordnet sein, der an dem proximalen Ende des Katheters in einen sich im Inneren des Katheters bis zu seinem distalen Ende erstreckenden Kanal einführbar ist.

Der elektromechanische Wandler besteht vorzugsweise aus einem piezoelektrischen Element, daß sich besonders einfach an die baulichen Abmessungen des Katheters anpassen läßt und elektrisch besonders einfach anzusteuern ist.

Eine besonders einfach zu realisierende Möglichkeit, den Katheter in eine oszillierende Bewegung quer zu seiner Längsausdehnung zu versetzen, wird dadurch erreicht, daß die Antriebsvorrichtung am proximalen Ende des Katheters angeordnet ist, daß an der Antriebsvorrichtung ein Steuerdraht angeschlossen ist, der sich durch einen Kanal im Inneren des Katheters hindurch bis zu dessen distalen Ende erstreckt und von der Antriebsvorrichtung in eine translatorische oder rotatorische Bewegung versetzt wird, und daß Mittel zur Umsetzung der translatorischen bzw. rotatorischen Bewegung des Steuerdrahts in eine oszillierende Bewegung vorgegebener Bereich des Katheters quer zu seiner Längsausdehnung vorgesehen sind. Dadurch wird erreicht, daß der Katheter gezielt an den vorbestimmten Stellen in die oszillierende Bewegung versetzt wird, an denen die Mittel zur Umsetzung der Bewegung des Steuerdrahtes in die Bewegung des Katheters vorgesehen sind.

Die Mittel zur Umsetzung der translatorischen bzw. rotatorischen Bewegung des Steuerdrahts in eine Bewegung des Katheters quer zu seiner Längsrichtung bestehen vorzugsweise aus vom geradlinigen Verlauf exzentrisch abweichenden Außenkonturen des Steuerdrahts. Solche, vom geradlinigen Verlauf abweichende Außenkonturen des Steuerdrahts können beispielsweise durch ansatzförmige Ausbildungen oder exzentrisch angeordnete bereichsweise Verdickungen des Steuerdrahts gebildet werden. Da der Katheter elastisch ist, folgt er in seinem Verlauf den Außenkonturen des Steuerdrahts, so daß bei Bewegung des Steuerdrahts relativ zum Katheter dieser in den Bereichen, in denen der Steuerdraht vom geradlinigen Verlauf abweicht, in eine Bewegung quer zu seiner Lenkungsausdehnung versetzt wird. Diese Querbewegung kann noch dadurch verstärkt werden, indem der Katheter in seinen Inneren mit schräg zu seiner Längsausdehnung verlaufenden Auflaufflächen für die exzentrischen Außenkonturen des Steuerdrahts versehen ist.

Vorzugsweise sind die vom geradlinigen Verlauf exzentrisch abweichenden Außenkonturen des Steuerdrahts in Form von Krümmungen des Steuerdrahts ausgebildet, wodurch eine einfachst mögliche Ausbildung des Steuerdrahts erreicht wird. Dabei wird zum Einführen des Steuerdrahts in das Innere des Katheters zunächst ein flexibles, im Vergleich zum Steuerdraht jedoch steiferes Rohr auf diesen aufgeschoben und das Rohr gemeinsam mit dem nun geradlinig im Rohr verlaufenden Steuerdraht in den Katheter eingeschoben. Wird daraufhin nur das Rohr wieder aus dem Katheter herausgezogen, so nimmt der Steuerdraht in dem Katheter seine gekrümmte Form an.

Zur Erläuterung der Erfindung wird im folgenden auf die Figuren der Zeichnung Bezug genommen; im einzelnen zeigen:
- FIG 1: einen Querschnitt durch das distale Ende eines Elektrodenkatheters mit einem elektromechanischen Wandler als integralen Bestandteil des Katheters,
- FIG 2: einen in das Innere eines Katheters einführbaren Steuerdraht mit einem elektromechanischen Wandler,
- FIG 3: A - D einen Elektrodenkatheter, einen mittels eines Rohrs in den Katheter einführbaren, einfach gekrümmten Steuerdraht und eine Antriebsvorrichtung zum Anschließen an den Steuerdraht im Bereich des proximalen Endes des Katheters und
- FIG 4: die Anordnung nach FIG 3 D mit einem mehrfach gekrümmten Steuerdraht.

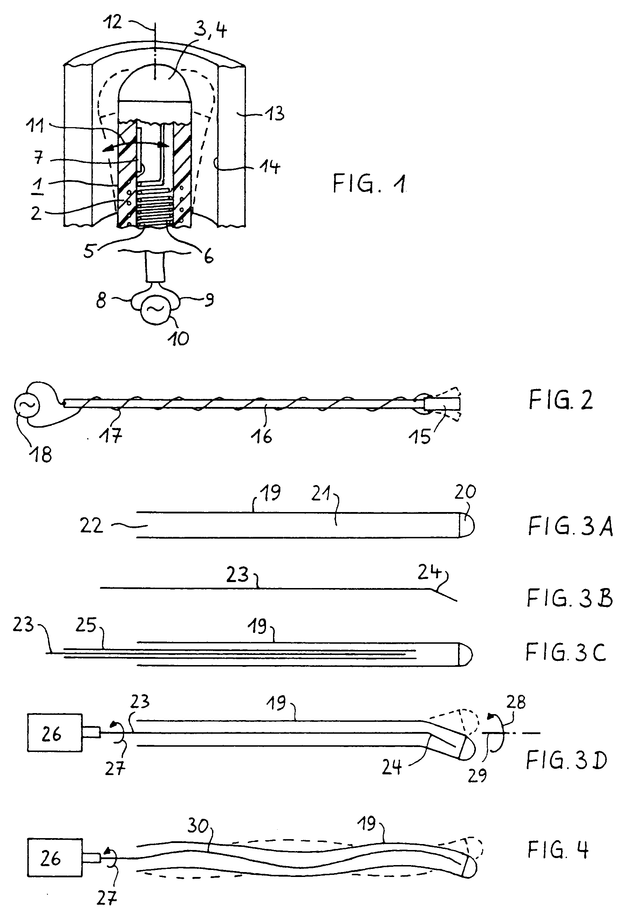

Figur 1 zeigt in stark vereinfachter Darstellung einen Querschnitt durch den distalen Endbereich eines Elektrodenkatheters 1. Der Elektrodenkatheter 1 besteht aus einem langgesteckten flexiblen Isolierschlauch 2, der an dem distalen Ende 3 einen Elektrodenkopf 4 trägt. An dem Elektrodenkopf 4 ist eine Elektrodenleitung 5 angeschlossen, die sich durch einen Kanal 6 im Inneren des Katheters 1 hindurch bis an sein hier nicht gezeigtes proximales Ende erstreckt und dort mit einem Anschlußelement verbunden ist. Im Bereich des distalen Endes 3 des Katheters 1 ist ein elektromechanischer Wandler 7 angeordnet, der als piezoelektrischer Biegewandler ausgebildet ist und an der Kanalwand im Inneren des Katheters 1 anliegt. Der elektromechanische Wandler 7 ist mit Zuleitungen 8 und 9 versehen, die am proximalen Ende des Katheters 1 an einem Wechselspannungsgenerator 10 angeschlossen sind. Der Wechselspannungsgenerator 10 beaufschlagt den elektromechanischen Wandler 7 mit einer Wechselspannung, die von dem Wandler 7 in eine oszillierende Biegebewegung 11 umgesetzt wird. Diese Biegebewegung 11 wird auf den flexliblen Isolierschlauch 2 des Katheters 1 übertragen und versetzt diesen in eine entsprechende, quer zur Längsausdehnung 12 des Katheters 1 erfolgende oszillierende Bewegung; die Längsausdehnung 12 des Katheters 1 ist hier durch seine Längsachse verdeutlicht. Durch die oszillierende Querbewegung 11 des Katheters 1 wird dieser ständig von der Innenwand 13 des ihn aufnehemenden Blutgefäßes 14 abgestoßen, wobei bei einer genügend hohen Frequenz der Katheter 1 quasi auf der Innenwand 14 des Blutgefäßes 13 schwebt.

Figur 2 zeigt als alternatives Ausführungbeispiel zu Figur 1 die Anordnung eines elektromechanischen Wandlers 15 am Ende eines Steuerdrahts 16, der vom proximalen Ende des Katheters her in den Kanal 6 (vgl. Figur 1) des Katheters 1 eingeschoben wird, so daß der elektromechanische Wandler 15 im Bereich des distalen Endes 3 des Katheters 1 zu liegen kommt. Der elektromechanische Wandler 15 ist mit Zuleitungen versehen, von denen eine von dem Steuerdraht 16 und die andere von einer gegenüber dem Steuerdraht 16 isolierten Leitung 17 gebildet ist. Die Ansteuerung des elektromechanischen Wandlers 15 erfolgt wie beim Beispiel nach Figur 1 durch einen Wechselspannungsgenerator 18.

Figur 3 A zeigt stark vereinfacht einen flexiblen Elektrodenkatheter 19 mit einem Elektrodenkopf 20 am distalen Ende und einem Kanal 21, der am proximalen Ende eine Öffnung 22 aufweist und sich von dort durch den Katheter 19 hindurch bis zu dessen distalen Ende hin erstreckt. In Figur 3 B ist ein ebenfalls flexibler Steuerdraht 23 dargestellt, der zum Einführen in den Katheter 19 vorgesehen ist und an seinem, im Bereich des distalen Endes des Katheters 19 zu liegen kommenden Ende eine vom geradlinigen Verlauf des Steuerdrahts 19 abweichende Krümmung 24 aufweist. Wie Figur 3 C zeigt, wird zum Einführen des Steuerdrahts 23 in den Katheter 19 zunächst ein ebenfalls flexibles, jedoch im Vergleich zum Steuerdraht 23 steiferes Rohr 25 auf diesen aufgeschoben, so daß der Steuerdraht 23 im Bereich seiner Krümmung 24 gestreckt wird. Anschließend wird der Steuerdraht 23 zusammen mit dem übergeschobenen Rohr 25 in den Katheter 19 eingeführt. Wird dann, wie in Figur 3 D gezeigt, das Rohr 25 aus den Katheter 19 wieder herausgezogen, so nimmt der Steuerdraht 23 an seinem Ende im Katheter 19 wieder die vorgegebene Krümmung 24 ein und beugt den Katheter 19 in entsprechender Weise Am proximalen Ende des Katheters 19 wird nun an dem Steuerdraht 23 eine Antriebsvorrichtung 26 bestehend aus einem Elektromotor angeschlossen, die den Steuerdraht 23 in ein, schnelle Rotationsbewegung 27 versetzt. Durch die Rotationsbewegung des Steuerdrahts 23 wird der Katheter 19 im Bereich der Krümmung 24 in eine quer zur Längsausdehnung des Katheters 19 erfolgende Kreisbewegung 28 um die Längsachse 29 des Katheters 19 herum versetzt, wobei der Katheter 19 im Unterschied zum Steuerdraht 23 jedoch nicht selbst rotiert.

Die in Figur 4 dargestellte Anordnung unterscheidet sich von der Anordnung nach Figur 3 D nur dadruch, daß der Steuerdraht 30 im Katheter 19 in Richtung seiner Längsausdehnung mehrfach gekrümmt ist, so daß der Katheter 19 in entsprechender Weise gebeugt wird. Dadurch wird erreicht, daß die von der Antriebsvorrichtung 26 auf den Steuerdraht 30 übertragene Rotationsbewegung 27 in eine wellenförmige Querbewegung des Katheters 19 umgesetzt wird. Dadurch wird nicht nur im Bereich des distalen Ende des Katheters 19 sondern auch entlang seiner Längsausdehnung die Reibung zwischen dem Katheter und dem ihn jeweils aufnehmenden Blutgefäß herabgesetzt.

## Patentansprüche

1. Elektrodenkatheter mit einer Anordnung zum intravaskulären Implantieren oder Explantieren des Elektrodenkatheters (1, 19), an dem eine Antriebsvorrichtung (7, 15, 26) angeordnet ist, die den Elektrodenkatheter (1, 19) in eine oszillierende Bewegung (11, 28) versetzt, **gekennzeichnet durch** Mittel (7, 15, 24) zur Erzeugung der oszillierende Bewegung (11, 28) nicht in Richtung der Längsausdehnung des Elektrodenkatheters sondern quer dazu.

2. Elektrodenkatheter nach Anspruch 1, **dadurch gekennzeichnet**, daß die Antriebsvorrichtung aus einem im Inneren des Elektrodenkatheters (1) angeordneten elektromechanischen Wandler (7,15) besteht.

3. Elektrodenkatheter nach Anspruch 2, **dadurch gekennzeichnet,** daß der elektromechanische Wandler (15) an einem Steuerdraht (16) angeordnet ist, der an dem proximalen Ende des Elektrodenkatheters (1) in einen sich im Inneren des Elektrodenkatheters (1) bis zu seinem distalen Ende (3) erstreckenden Kanal (6) einführbar ist.

4. Elektrodenkatheter nach Anspruch 2 oder 3, **dadurch gekennzeichnet,** daß der elektromechanische Wandler (7,15) aus einem piezoelektrischen Element besteht.

5. Elektrodenkatheter nach Anspruch 1, **dadurch gekennzeichnet**, daß die Antriebsvorrichtung (26) am proximalen Ende des Elektrodenkatheters (19) angeordnet ist, daß an der Antriebsvorrichtung (26) ein Steurdraht (23) angeschlossen ist, der sich durch einen Kanal (21) im inneren des Elektrodenkatheters (19) hindurch bis zu dessen distalen Ende erstrecht und von der Antriebsvorrichtung (26) in eine translatorische oder rotatorische Bewegung (27) versetzt wird, und das Mittel (24) zur Umsetzung der translatorischen bzw. rotatorischen Bewegung (27) des Steuerdrahts (23) in eine oszillierende Bewegung (28) vorgegebener Bereiche des Elektrodenkatheters (19) quer zu seiner Längsausdehnung (29) vorgesehen sind.

6. Elektrodenkatheter nach Anspruch 5, **dadurch gekennzeichnet,** daß die Mittel (24) zur Umsetzung der translatorischen bzw. rotatorischen Bewegung (27) des Steuerdrahts (23) in eine Bewegung (28) des Elektrodenkatheters (19) quer zu seiner Längsausdehung (29) aus vom geradlinigen Verlauf exzentrisch abweichenden Außenkonturen des Steuerdrahts (23) bestehen.

7. Elektrodenkatheter nach Anspruch 6, **dadurch gekennzeichnet,** daß die vom geradlinigen Verlauf exzentrisch abweichenden Außenkonturen des Steuerdrahts (23) in Form von Krümmungen (24) des Steuerdrahts (23) ausgebildet sind.

## Claims

1. Electrode catheter having an arrangement for the intravascular implantation or explantation of the electrode catheter (1, 19) on which a driving device (7, 15, 26) is arranged that sets the electrode catheter (1, 19) in oscillatory motion (11, 28), characterised by means (7, 15, 24) for producing the oscillatory motion (11, 28) not in the direction of the longitudinal extent of the electrode catheter, but transversely thereto.

2. Electrode catheter according to claim 1, characterised in that the driving device consists of an electromechanical transducer (7, 15) that is arranged in the interior of the electrode catheter (1).

3. Electrode catheter according to claim 2, characterised in that the electromechanical transducer (15) is arranged on a control wire (16) which can be introduced, at the proximal end of the electrode catheter (1), into a channel (6) that extends within the interior of the electrode catheter (1) as far as its distal end (3).

4. Electrode catheter according to claim 2 or 3, characterised in that the electromechanical transducer (7, 15) consists of a piezoelectric element.

5. Electrode catheter according to claim 1, characterised in that the driving device (26) is arranged at the proximal end of the electrode catheter (19), in that connected to the driving device (26) there is a control wire (23) which extends through a channel (21) in the interior of the electrode catheter (19) as far as the latter's distal end and is set in translatory or rotatory motion (27) by the driving device (26), and in that means (24) are provided for converting the translatory or rotatory motion (27) of the control wire (23) into an oscillatory motion (28) of given regions of the electrode catheter (19) that is directed transversely in relation to the latter's longitudinal extent (29).

6. Electrode catheter according to claim 5, characterised in that the means (24) for converting the translatory or rotatory motion (27) of the control wire (23) into a motion (28) of the electrode catheter (19) that is directed transversely in relation to the latter's longitudinal extent (29) consist of outer contours of the control wire (23) that deviate eccentrically from the linear course.

7. Electrode catheter according to claim 6, characterised in that the outer contours of the control wire (23) that deviate eccentrically from the linear course are in the form of curvatures (24) of the control wire (23).

## Revendications

1. Cathéter à électrodes comportant un dispositif pour l'implantation ou l'extraction intravasculaire du cathéter à électrodes (1, 19), sur lequel il est prévu un dispositif d'entraînement (7, 15, 26) qui imprime un mouvement oscillant (11, 28) au cathéter à électrodes (1, 19), caractérisé par des moyens (7, 15, 24) pour générer le mouvement oscillant (11, 28) non pas dans la direction longitudinale du cathéter à électrodes mais transversalement à celle-ci.

2. Cathéter à électrodes selon la revendication 1, caractérisé par le fait que le dispositif d'entraînement comprend un transducteur électromécanique (7, 15) qui est disposé à l'intérieur du cathéter à électrodes (1).

3. Cathéter à électrodes selon la revendication 2, caractérisé par le fait que le transducteur électro-mécanique (15) est disposé sur un fil de commande (16) qui peut être introduit côté extrémité proximale du cathéter à électrodes (1) dans un canal (6) qui s'étend à l'intérieur dudit cathéter à électrodes (1), jusqu'à l'extrémité distale (3) de celui-ci.

4. Cathéter à électrodes selon la revendication 2 ou 3, caractérisé par le fait que le transducteur électro-mécanique (7, 15) est formé d'un élément piézoélectrique.

5. Cathéter à électrodes selon la revendication 1, caractérisé par le fait que le dispositif d'entraînement (26) est disposé à l'extrémité proximale du cathéter à électrodes (19), par le fait qu'un fil de commande (23) est connecté au dispositif d'entraînement (26), lequel fil de commande s'étend à travers un canal (21) à l'intérieur du cathéter à électrodes (19), jusqu'à l'extrémité distale de ce celui-ci et est mis en un mouvement de translation ou de rotation (27) par le dispositif d'entraînement,et par le fait que des moyens (24) sont prévus pour transformer le mouvement (27) de translation ou de rotation du fil de commande (23) en un mouvement oscillant (28) de zones prédéterminées du cathéter à électrodes (19) transversalement à sa direction longitudinale (29).

6. Cathéter à électrodes selon la revendication 5, caractérisé par le fait que les moyens (24) pour transformer le mouvement (27) de translation ou de rotation du fil de commande (23) en un mouvement (28) du cathéter à électrodes (19) transversalement à sa direction longitudinale (29) sont formés par des contours extérieurs du fil de commande (23) qui s'écartent de manière excentrée de la forme rectiligne.

7. Cathéter à électrodes selon la revendication 6, caractérisé par le fait que les contours extérieurs du fil de commande (23) qui s'écartent de manière excentrée de la forme rectiligne sont réalisés sous la forme de courbures (24) du fil de commande (23).
